# EUROPEAN PATENT SPECIFICATION

(11) **EP 3 215 232 B1**
(45) Date of publication and mention of the grant of the patent: **04.03.2020**
(21) Application number: 15785112.2
(22) Date of filing: 27.10.2015
(51) Int. Cl.: A61Q 13/00, A61Q 15/00, A61K 8/84, A61K 8/11, C11D 3/50

(54) **IMPROVEMENTS IN OR RELATING TO ORGANIC COMPOUNDS**
VERBESSERUNGEN AN ODER IM ZUSAMMENHANG MIT ORGANISCHEN VERBINDUNGEN
PERFECTIONNEMENTS APPORTÉS À DES COMPOSÉS ORGANIQUES OU EN RELATION AVEC CES DERNIERS

(30) Priority: 07.11.2014 EP 14290339
(43) Date of publication of application: 13.09.2017
(73) Proprietor: Givaudan S.A., 1214 Vernier (CH)
(72) Inventor: AUSSANT, Emmanuel, 75011 Paris (FR); FADEL, Addi, Metuchen NJ 08840 (US); HARRISON, Ian Michael, 78300 Poissy (FR); QUELLET, Christian, 2502 Bienne (CH); BURAKOWSKA-MEISE, Ewelina, 64686 Reichenbach Lautertal (DE); DENUELL, Wolfgang, 68163 Mannheim (DE); SOLTYS, Thomas, 67071 Ludwigshafen (DE)
(74) Representative: Global Patents
(86) International application number: PCT/EP2015/074810
(87) International publication number: WO 2016/071149

(56) References cited:
- EP-A1- 2 179 719
- WO-A1-03/066209
- WO-A1-2011/154893
- WO-A1-2011/161229
- WO-A1-2012/107323
- WO-A1-2013/092958
- WO-A2-01/94001
- US-A1- 2011 071 064

## Description

The present invention is concerned with encapsulated perfume compositions, comprising one or more core-shell capsules, wherein the core contains a perfume and the shell contains a polyurea resin ("polyurea capsules"). The invention also relates to consumer products containing said compositions.

Encapsulated perfume compositions are known in the art. They may be formed by a process of coating small solid particles or liquid droplets in a thin film of shell material. Although virtually any coating material, conceptually at least, is a candidate capsule shell material, in practice for commercial and regulatory reasons, to-date, there are relatively few materials that have been used in commercial products. Capsule shell material selection is determined by a number of factors including cost, availability, processing ease, and inherent barrier properties. Defining an optimal shell material for a given application can be complex since many interacting parameters determine success of a given capsule shell material.

It is known in the art to encapsulate perfume compositions in polyurea capsules. Encapsulated perfume compositions based on polyurea capsules can be produced by polyaddition of amine and isocyanate monomers under conditions described in the art, see for example WO 2011/161229 A1.

Encapsulated perfume compositions are typically prepared in the form of aqueous slurries. It is important to ensure that the perfume-containing capsules are well dispersed in the slurry, and it is particularly important to avoid phase separation of the capsules from the aqueous dispersing medium, in order to prevent creaming, sedimentation or coagulation. In order to properly disperse and suspend capsules within an aqueous dispersing medium, stably over time, dispersing aids may be employed in the manufacture of slurries.

A wide variety of dispersing aids are known in the art, and include polysaccharides, pectine, alginate, arabinogalactan, carageenan, gellan gum, xanthan gum, guar gum, acrylates/acrylic polymers, starches, water-swellable clays, acrylate/aminoacrylate copolymers, and mixtures thereof, maltodextrin; natural gums such as alginate esters; gelatine, protein hydrolysates and their quaternized forms; synthetic polymers and copolymers, such as poly(vinyl pyrrolidone-co-vinyl acetate), poly(vinyl alcohol-co-vinyl acetate), poly(maleic acid), poly(alkyleneoxide), poly(vinylmethylether), poly(vinylether-co-maleic anhydride), and the like, as well as poly-(ethyleneimine), poly((meth)acrylamide), poly(alkyleneoxide-co-dimethylsiloxane), poly(amino dimethylsiloxane), and the like.

Despite the variety of dispersing aids that are available for use, the selection of the appropriate dispersing aid, will depend on a number of factors, including the capsule shell chemistry, its morphology, its size and density, as well as the composition of the aqueous dispersing media, such as its pH and electrolyte content, all of which will be influenced to a certain extent by the encapsulation process conditions.

Indeed, it proved difficult to prepare in a reliable and reproducible way, encapsulated perfume compositions comprising polyurea capsules in the form of aqueous slurries. Phase separation, as well as slurry viscosity was difficult to control. When slurry viscosity is too high, often excessive processing forces need to be used to manipulate it, which in turn can damage the capsules. Furthermore, highly viscous slurries can be difficult to handle and can lead to difficulties when incorporating encapsulated perfume compositions into consumer product bases.

The applicant has now found, during the course of research leading to the present invention that by employing hydroxyethylcellulose as a dispersing aid, it was possible to form, in a straightforward manner, an encapsulated perfume composition, in the form of a slurry in which polyurea capsules were stably dispersed, and which possessed an acceptable viscosity.

Therefore, the invention provides in a first aspect an encapsulated perfume composition comprising one or more polyurea capsules, wherein the core contains a perfume, and wherein the capsules are in the form of a stable suspension in an aqueous medium having a viscosity of up to 3000 mPa.s and more particularly 150 to 3000 mPa.s when measured on a rheometer, for example a RheoStress™ 1 instrument (ThermoScientific), using rotating disks at a shear rate of 21 s⁻¹ at a temperature of 25 ° C, and wherein the aqueous dispersing medium contains a hydroxy ethyl cellulose dispersing aid.

As used hereinabove, the term "stable suspension" is intended to mean a suspension of the polyurea capsules, which upon visible inspection, shows no sign of phase separation, such as creaming, settling, precipitation or coagulation when stored for a period of 2 weeks at a temperature of 50 °C.

Any hydroxyethylcellulose that is suitable for use in consumer products may be employed as a dispersing aid in accordance with the present invention. Preferred grades, however, are those suitable for use in cosmetics. Particularly preferred grades of hydroxyethyl cellulose include those Natrosol™ products known in the art, and particularly Natrosol™ 250 HX.

In a particular embodiment of the invention, the amount of hydroxyethyl cellulose employed in a slurry is 0.05 to 1.0 %, more particularly 0.05 to 0.5 % by weight based on the total weight of the slurry.

Provided hydroxyethyl cellulose is employed as a dispersing aid, additional dispersing aids may also be employed, if desired. Examples of suitable additional dispersing aids include any of those mentioned herein above. In particular, said additional dispersing aids include starches, for example National 465, Purity W, or starch B990; or acrylate polymer or copolymers, for example Tinovis CD, Ultragel 300 and Rheocare TTA.

When additional dispersing aids are employed, they may be used in amounts in the range of 0.1 to 5.0 %, more particularly 0.5 to 4 % by weight and still more particularly 1 to 3% by weight, based on the weight of the slurry.

The hydroxyethyl cellulose is preferably added to the slurry once it is formed. Adding hydroxyethyl cellulose during the formation of the capsules is preferably avoided because it may increase slurry viscosity during capsule preparation and be detrimental to capsule formation.

The encapsulated perfume composition according to the present invention may be prepared by any method known in the art for producing capsules by interfacial polyaddition of an amine with an isocyanate.

Representative preparative methods are disclosed in WO 2011/161229 A1 and WO 2011/160733 A1. According to WO 2011/161229 A1 or WO 2011/160733 A1 the polyurea microcapsules are prepared in presence of polyvinylpyrrolidone (PVP) as a protective colloid.

WO 2012/107323 A1 discloses polyurea microcapsules having a polyurea shell comprising the reaction product of a polyisocyanate with guanazole and an amino acid in presence of anionic stabilizers or surfactants like anionic polyvinyl alcohol, such as Mowiol® KL-506 sold by Kuraray.

EP 0 537 467 describes microcapsules prepared from isocyanates which are containing polyethylenoxide groups, in the presence of stabilizers like polyvinyl alcohol, e.g. partially or totally saponified polyvinyl acetate.

WO 2007/096592 A1 described a microencapsulation process in which an oil phase is emulsified in a continuous aqueous phase, generally stabilized by a surfactant system like polyvinyl alcohols or carboxylated and sulphonated derivatives thereof.

In a typical procedure, the encapsulated perfume composition can be prepared according to a procedure in which an aqueous phase is prepared containing a surfactant and/or a protective colloid such as those described below. The aqueous phase is stirred vigorously for a time period of only a few seconds up to a few minutes. A hydrophobic phase may then be added to the aqueous phase. The hydrophobic phase will contain the perfume to be encapsulated, and an isocyanate. The hydrophobic phase may also include suitable solvents, although, in a preferred aspect of the present invention, no solvents are employed. After a period of vigorous stirring, an emulsion is obtained, in which the hydrophobic phase is dispersed as tiny droplets in the aqueous continuous phase. The rate of stirring may be adjusted to influence the size of droplets of hydrophobic phase in the aqueous phase.

An aqueous solution containing the amine is then added to initiate the polyaddition reaction. The amount of amine which is introduced is usually in excess, relative to the stoichiometric amount needed to convert the free isocyanate groups.

The polyaddition reaction proceeds generally at a temperature ranging from approximately 0 to 100 degrees centigrade, for a period of time ranging from a few minutes to several hours.

Conditions for creating capsules by interfacial polyaddition are well known in the art and no further elaboration of those conditions, which are within the purview of the skilled person, is needed here. Specific description relating to the preparation of the capsules is provided in the examples below.

Amines useful in the formation of capsules include those compounds containing one or more primary or secondary amine groups, which can react with isocyanates to form polyurea. When the amine contains only one amino group, the compound will contain one or more additional functional groups that would form a network through a polymerisation reaction.

Examples of suitable amines include 1,2-ethylenediamine, 1,3-diaminopropane, 1,4-diaminobutane, 1,6-diaminohexane, hydrazine, 1,4-diaminocyciohexane and 1,3-diamino-1-methylpropane, diethylenetriamine, triethylenetetramine and bis(2-methylaminoethyl)methylamine.

Other useful amines include poly ethyieneamine (CH₂CH₂NH)ₙ such as ethyleneamine, diethyleneamine, ethylenediamine, triethylenetetramine, tetraethylenepentamine; polyvinylamine (CH₂CHNH₂)n sold by BASF (Lupamine different grades); poly ethyleneimine (CH₂CH₂N)ₓ-(CH₂CH₂NH)_{y}-(CH₂CH₂NH₂)_{z} sold by BASF under Lupasol™ grades; polyetheramine (Jeffamine from Huntsman); guanidine, guanidine salt, melamine, hydrazine and urea.

A particularly preferred amine is a polyethyleneimine (PEI), more particularly a PEI from the Lupasol™ range supplied by BASF, still more particularly Lupasol™PR8515.

Isocyanates useful in the formation of polyurea microcapsules include di- and tri-functionalised isocyanates such as 1,6-diisocyanatohexane, 1,5-diisocyanato-2-methylpentane, 1,5-diisocyanato-3-methylpentane, 1,4-diisocyanato-2,3-dimethylbutane, 2-ethyl-1,4-diisocyanatobutane, 1,5-diisocyanatopentane, 1,4- diisocyanatobutane, 1,3-diisocyanatopropane, 1,10-diisocyanatodecane, 1,2-diisocyanatocyclobutane, bis(4-isocyanatocyclohexyl)methane, or 3,3,5-trimethyl-5- isocyanatomethyl-1-isocyanatocyclohexane.

Other useful isocyanates include also the oligomers based on those isocyanate monomers, such as homopolymer of 1,6-diisocyanatohexane. All those monomers and olligomers are sold under the trade name Desmodur by Bayer. Also included are the modified isocyanates and in particular, the waterdispersible isocyanate such as Hydrophilic Aliphatic Polyisocyanate based on Hexamethylene Diisocyanate, (sold under the name BAYHYDUR™).

The classes of protective colloid or emulsifier, which may be employed include maleic-vinyl copolymers such as the copolymers of vinyl ethers with maleic anhydride or acid, sodium lignosulfonates, maleic anhydride/styrene copolymers, ethylene/maleic anhydride copolymers, and copolymers of propylene oxide, ethylenediamine and ethylene oxide, polyvinylpyrrolidone, polyvinyl alcohols, fatty acid esters of polyoxyethylenated sorbitol and sodium dodecylsulfate. Polyvinyl alcohols are particularly preferred. Particularly preferred polyvinyl alcohols are the G-polymer type available from Nichigo.

Particular protective colloids include polyvinyl alcohol copolymers having a degree of hydrolysis in the range of 85 to 99.9 %. As used herein, the term "polyvinyl alcohol copolymer" means a polymer of vinyl alcohol/vinyl acetate with comonomers.

It is known that polyvinyl alcohol is produced by hydrolysis (deacetylation) of polyvinyl acetate, whereby ester groups of polyvinyl acetate are hydrolysed into hydroxyl groups, thus forming polyvinyl alcohol.

The degree of hydrolysis reflects the percentage of groups that are converted by hydrolysis. The term "polyvinyl alcohol" qualified by a degree of hydrolysis, means therefore, a vinyl polymer containing both ester and hydroxyl groups.

In a particular embodiment of the invention, copolymers of polyvinyl alcohol with a degree of hydrolysis in the range of 85 to 99.9 %, more particularly 85 to 95 % may be used as protective colloids.

The degree of hydrolysis can be determined by techniques well known in the art, for example, according to DIN 53401.

The polyvinyl alcohol copolymers contain addition comonomers, that is, comonomers that are polymerized with a vinyl ester in a first step, followed by hydrolysis of the ester groups to form the copolymer of polyvinyl alcohol in a second step. Copolymers may be formed by radical polymerization of vinyl acetate and comonomers in a manner known per se.

Polyvinyl alcohol copolymers may contain unsaturated hydrocarbons as comonomers. These hydrocarbons may be modified with charged or non-charged functional groups. Particular comonomers include, but are not limited to:
- unsaturated hydrocarbons with 2 or 3 carbon atoms and no functional groups, e.g. ethylene;
- unsaturated hydrocarbons having 2 to 6 carbon atoms and non-charged functional groups, such as hydroxyl groups, e.g. buten-1,4-diol;
- unsaturated hydrocarbons having anionic groups, such as carboxyl, and/or sulphonic acid groups;
- unsaturated hydrocarbons having cationic groups, such as quaternary ammonium groups.

Particular copolymers of polyvinyl alcohol include those having a degree of hydrolysis of 85 to 99.9 %, and more particularly 85 to 95 %; and which contain:
- 0.1 to 30 mol % of comonomers containing anionic groups as mentioned above; or
- 0.1 to 30 mol % of comonomers containing cationic groups as mentioned above; or
- 0.1 to 30 mol % of comonomers with unsaturated hydrocarbons having 2 to 6 carbon atoms and non-charged functional groups, especially two hydroxyl groups,
wherein mol % is based on the vinyl acetate/comonomer polymerization mixture.

Suitable copolymers of polyvinyl alcohol and comonomers having 1,2-diol structure are described in EP 2 426 172 and EP 2 648 211.

The following protective colloids are particularly useful in the preparation of polyurea capsule compositions of the present invention:
- Anionic polyvinyl alcohol copolymers with a degree of hydrolysis of greater than 80 %, preferably 85.0 % to 995 %, and a viscosity of 2 mPas to 70 mPas (DP 100-6000), for example K-polymer KL-318 from Kuraray (viscosity 20-30 mPas, hydrolysis 85.0 to 90.0 %); Gohsenal T-350 from Nippon Gohesi (viscosity 27-33 mPas, hydrolysis 93.0 to 95.0 %); Gohseran L-3266 from Nippon Gohsei (viscosity 2.3 to 2.7 mPas, hydrolysis 86.5 to 89.0 %)
- Non-charged polyvinyl alcohol copolymers with a degree of hydrolysis of greater that 80 %, preferably 85.0 to 99.5 %, and a viscosity of 2 mPas to 70 mPas (DP 100-6000), for example G-polymer OKS-8041 from Nippon Gohsei (viscosity 2.8 to 3.3 mPas, hydrolysis 88.0 to 90.0 %), G-polymer AZF-8035 from Nippon Gohsei (viscosity 2.8 to 3.3 mPas, hydrolysis 98.5 to 99.5 %); and
- Cationic polyvinyl alcohol copolymers with a degree of hydrolysis of greater than 80 %, and more particularly 85.0 to 99.5 %, and a viscosity of 2 mPas to 70 mPas (DP 100-6000), for example Gohsefimer K-210 from Nippon Gohsei (viscosity 18.0 to 22.0 mPas, hydrolysis 85.5 to 88.0 %).

The protective colloid may or may not be a constituent of the capsule shell. Generally, the total amount of protective colloid expressed as a percentage by weight based on the weight of the slurry is in the range of 0.1 to 20%, more particularly 1% to 10% and still more particularly 1.5% to 5% by weight.

Combinations of two or more different protective colloids may also be employed in the present invention.

If the encapsulated perfume composition of the present invention is intended to be stored in the form of a slurry, the pH of the slurry is adjusted to a level of 5 to 10. In an alkaline slurry, this may be achieved with the addition of a suitable acid, such as citric acid or formic acid.

Furthermore, a preservative is typically added to the slurry in order to prevent microbial contamination the encapsulated perfume composition of the present invention may contain a preservative. The preservative may be encapsulated and/or it may be contained in the aqueous suspending medium of the slurry. Suitable preservatives include quaternary compounds, biguanide compounds, and mixtures thereof. Non-limiting examples of quaternary compounds include benzalkonium chlorides and/or substituted benzalkonium chlorides such as commercially available Barquat® (available from Lonza), Maquat® (available from Mason), Variquat® (available from Witco/Sherex), and Hyamine® (available from Lonza); di(C6-C14)alkyl di short chain (C1-4 alkyl and/or hydroxyalkl) quaternary such as Bardac® products of Lonza; N-(3-chloroallyl) hexaminium chlorides such as Dowicide® and Dowicil® available from Dow; benzethonium chloride such as Hyamine® from Rohm & Haas; methylbenzethonium chloride represented by Hyamine® 10* supplied by Rohm & Haas, cetylpyridinium chloride such as Cepacol chloride available from of Merrell Labs; and diester quaternary ammonium compounds. Examples of preferred dialkyl quaternary compounds are di(C8-C12)dialkyl dimethyl ammonium chloride, such as didecyldimethylammonium chloride (Bardac® 22), and dioctyldimethylammonium chloride (Bardac® 2050). The quaternary compounds useful as cationic preservatives and/or antimicrobial agents herein are preferably selected from the group consisting of dialkyldimethylammonium chlorides, alkyldimethylbenzylammonium chlorides, dialkylmethylbenzylammonium chlorides, and mixtures thereof. Other preferred cationic antimicrobial actives useful herein include diisobutylphenoxyethoxyethyl dimethylbenzylammonium chloride (commercially available under the trade name Hyamine® 1622 from Rohm & Haas) and (methyl)diisobutylphenoxyethoxyethyl dimethylbenzylammonium chloride (i.e. methylbenzethonium chloride).

The encapsulated perfume composition may contain surfactants. Surfactants include nonionic, cationic, anionic and zwitterionic varieties.

In addition to encapsulated perfume, the slurry may contain non-encapsulated, i.e. free perfume, external of the capsules in the aqueous carrier medium.

The process described hereinabove is a convenient and versatile means for preparing encapsulated perfume compositions of the present invention. The encapsulated perfume compositions can be prepared containing polyurea capsules having a wide range of dimensions. Encapsulated perfume compositions according to the present invention may comprise capsules having a volume average capsule diameter of 20 to 250 microns, more particularly 20 to 90 microns, still more particularly 20 to 75 microns, and more particularly still 30 to 50 microns.

As used herein, the volume average particle size is measured by light scattering measurements using a Malvern 2000S instrument and the Mie scattering theory. The principle of the Mie theory and how light scattering can be used to measure capsule size can be found, for example H. C. van de Hulst, Light scattering by small particles. Dover, New York, 1981. The primary information provided by static light scattering is the angular dependence of the light scattering intensity, which in turn is linked to the size and shape of the capsules. However, in a standard operation method, the size of a sphere having a size equivalent to the size of the diffracting object, whatever the shape of this object, is calculated by the Malvern proprietary software provided with the apparatus. In case of polydisperse samples, the angular dependence of the overall scattering intensity contains information about the size distribution in the sample. The output is a histogram representing the total volume of capsules belonging to a given size class as a function of the capsule size, whereas an arbitrary number of 50 size classes is typically chosen.

Experimentally, a few drops of slurry containing about 10% of capsules are added to a circulating stream of degased water flowing through a scattering cell. The angular distribution of the scattering intensity is measured and analyzed by Malvern proprietary software to provide the average size and size-distribution of the capsules present in the sample. In the context of the present invention the percentiles Dv 10, Dv 50 and Dv 90 are used as characteristics of the capsule size distribution, whereas Dv 50 corresponds to the median of the distribution.

The shell weight, expressed as a percentage of the total weight of the polyurea capsules (core material + shell material), is an important parameter in determining both the stability the performance of the polyurea capsules.

Applicant has found that polyurea capsules can be difficult to produce with highly uniform shell thickness.

Polyurea capsules are formed by a process of interfacial polymerization. An oil-in-water emulsion is prepared and the shell-forming materials are contained in both the dispersed oil phase and the continuous aqueous phase. In order for shell-formation to take place, shell-forming material must diffuse through two different phases in order to reach the oil-water interface before reacting to form the capsule shell. The shell properties or characteristics will be directly affected by the composition of the oil phase, which in the case of a perfume oil, will typically contain tens or even hundreds of different perfume ingredients, each having its own physical and chemical properties (such as solubility and partition coefficient). The rate at which a shell-forming material will be able to diffuse towards the oil-water interface will vary depending on the composition of the complex perfume oil. As a result, shell morphology, in particular shell thickness uniformity, may be difficult to control precisely. As such, shell thickness may be an unreliable parameter, which does not correlate well with capsule performance.

This may be contrasted, for example, with core-shell capsules made by a process of complex coacervation (gelatin capsules, for example). In such a process, colloids are caused to coacervate around oil droplets dispersed in an external aqueous phase. However, unlike the process used in the formation of polyurea capsules, all the shell-forming material is contained in a single phase (the external aqueous phase) and only has to migrate to the oil-water interface through this phase. Furthermore, these capsules are typically formed around droplets of a sacrificial oil or solvent having a very high clogP. Only once the capsules are formed are they then immersed in a perfume composition, which diffuses into the capsule cores to displace the oil/solvent. This coacervation process promotes the formation of regularly-shaped capsules with uniform shell thickness.

Accordingly, applicant found that shell weight is a more reliable parameter than shell thickness for the purpose of controlling the quality of polyurea capsules. Shell weight can be manipulated in a straightforward manner by controlling the amount of shell-forming monomers added during the encapsulation process.

In a particular embodiment of the present invention, the shell weight of polyurea capsules, expressed as a percentage of the total weight of the capsules (encapsulated material + shell material), is 5 % to 40 %, still more particularly 10 % to 25 % and still more particularly 12 % to 20 %.

The relationship of shell weight to the volume average diameter of the capsules is also important in determining the release characteristics of the encapsulated perfume composition.

More particularly, applicant found that breakable capsules could be formed that were sufficiently mechanically robust such that when not subjected to compression or shear forces, they provide very little perfume impression, but release perfume in response to vigorous mechanical agitation. Applicant found that this could be achieved if the ratio of the shell weight (expressed as a percentage of the total weight of the capsules: encapsulated material + shell material) to the capsule diameter (expressed in microns) is 0.7 microns⁻¹ or less, more particularly 0.6 microns⁻¹ or less, and still more particularly 0.2 microns⁻¹ or less. Capsules characterized by this ratio are particularly suitable for incorporation into leave-on products, such as deodorants and antiperspirants, wherein, upon application, they can release perfume in response to frictional contact between skin and skin or clothing.

In an embodiment of the invention, the nominal rupture stress of polyurea capsules, expressed in MPa is in the range of 0.1 to 2 MPa, more particularly 0.2 MPa to 1.5 MPa, and still more particularly 0.4 MPa to 1 MPa.

The nominal rupture stress can be measured by the micro-manipulation technique, which is known in the art. The capsules are diluted in distilled water and dried on a microscope stage for 30 minutes at room temperature (24±1°C). The principle of the micro-manipulation technique is to compress a single capsule between two parallel surfaces. A single capsule is compressed and held, compressed and released, and compressed to large deformations or rupture at a pre-set speed of 1 micrometer per second. Simultaneously, the force being imposed on them and their deformation can be determined. The technique uses a fine probe positioned perpendicular to the surface of the capsule sample. The probe is connected to a force transducer, which is mounted on a 3-dimensional micro-manipulator that can be programmed to travel at a given speed. The whole process is carried out on an inverted microscope. From the curve of force versus sampling time, the relationship between the force and the capsule deformation to bursting, and its initial diameter are obtained. The technique of micro-manipulation is more fully explained in Zhang, Z., Saunders, R. and Thomas, C. R., Micromanipulation measurements of the bursting strength of single microcapsules, Journal of Microencapsulation 16(1), 117-124 (1999). The force at capsule rupture expressed in force units (Newton), which is then converted to rupture stress, expressed in pressure units (Pascal), through dividing the rupture force by the cross-sectional area of the capsule. The tip, or probe, used for the micro-manipulation should be approximately the same size as the capsules, and is typically between 10-50 microns. Typically, the force at rupture is measured on single capsules and repeated over typically 50 capsules and the average value is used to calculate the nominal rupture stress according to the present invention.

Capsule loading is determined by varying the proportion of shell-forming material and core-forming material employed in the encapsulation process. High levels of perfume may be encapsulated within an encapsulated perfume composition of the present invention.

In a particular embodiment of the present invention, the amount of capsules (encapsulated material + shell material) in the slurry is in the range of 5% to 75%, more particularly 25% to 50%, and still more particularly 30% to 40% by weight based on the weight of the slurry.

Furthermore, the total amount of encapsulated perfume expressed as a percentage by weight based on the weight of the slurry is in the range of 10% to 50%, more particularly 20% to 40% and still more particularly 25 to 35% by weight.

Furthermore, high loadings of perfume can be encapsulated despite the relatively low shell weight. Indeed, in another aspect of the present invention, the ratio of total encapsulated perfume to the shell material may range from 60% to 95% by weight, more particularly 75% to 80% and still more particularly 80% to 88% by weight.

The core-shell weight ratio may be obtained by weighing an amount of capsules that have been previously washed with water and separated by filtration. The core is then extracted by solvent extraction techniques to give a core weight. The shell weight is obtained from simple mass balance taking into account the initial amount of encapsulating materials in weight %.

The capsule cores are filled with perfume oil. The perfume oil is composed of one or more perfume ingredients. In general terms, perfume ingredients will belong to chemical classes as varied as alcohols, ketones, esters, ethers, acetates, terpene hydrocarbons, nitrogenous or sulphurous heterocyclic compounds and essential oils, which can be of natural or synthetic origin. Many of these perfume ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery.

As is generally known in the art, perfume retention during capsule formation, as well as stability towards leakage once a capsule is formed, is promoted through the use of high amounts of perfume ingredients having a relatively high C log P. In particular, at least 50 %, more particularly more than 60 %, and still more particularly more than 80 % of ingredients should have a C log P of 2.5 or greater, and more particularly 3.3 or greater, and still more particularly 4.0 or greater. Use of such perfume ingredients is generally regarded as helpful in reducing diffusion of perfume through a capsule shell and into a product base under specific time, temperature, and concentration conditions.

The values of C log P of perfume ingredients have been reported in many databases, including the Pomona 92 database, available from Daylight Chemical Information Systems, Inc., Daylight CIS, Irvine, California.

It is common to use solvents in admixture with the perfume ingredients. Solvent materials are hydrophobic materials that are miscible in the perfume ingredients, and which have little or no odour in the quantities employed. Solvents commonly employed have high C log P values, for example greater than 6 and even greater than 10. Solvents include triglyceride oil, mono and diglycerides, mineral oil, silicone oil, diethyl phthalate, polyalpa olefins, castor oil and isopropyl myristate.

US 2011/071064 A1 is concerned with polyurea capsules for use in personal care applications. It is particularly concerned with means of manipulating the shell properties in order to manipulate the release profile of the capsules. It is stated therein, that a solvent should be employed in the core in an amount of greater than 10 %, more particularly greater than 30 %, and still more particularly greater than 70 % by weight.

The applicant surprisingly found that it is possible to employ substantially no solvent material in the core. Indeed, applicant found that it is possible to prepare encapsulated perfume compositions wherein the encapsulated cores are composed entirely of perfume ingredients and no solvents. Solvent-free encapsulated perfume compositions may be employed, in particular, when the perfume ingredients making up the core material have limited water solubility. In particular, the core material preferably should be formed of a large proportion of perfume ingredients having a solubility in water of 15,000 ppm or less, more particularly 5,000 ppm or less, and still more particularly 3,000 ppm or less. More particularly, at least 60 %, more particularly at least 70 % and still more particularly at least 80 % of perfume ingredients should have a solubility in water of 15,000 ppm or less, more particularly 5,000 ppm or less, and still more particularly 3,000 ppm or less.

Avoiding the use of a solvent in the capsule cores is generally advantageous in terms of cost and the environment. But more particularly, in relation to leave-on products, if one is able to prepare capsules with high perfume loading by avoiding the use of solvents, one can prepare encapsulated perfume compositions with lower levels of capsules. The lower the amount of capsules employed, the less likelihood there is of visible residue being deposited on garments in intimate contact with a subject's skin.

Whereas the encapsulated perfume composition of the present invention may be employed to encapsulate any perfume ingredients, the applicant found that there can be difficulties associated with the encapsulation of perfume ingredients possessing aldehyde functionality. More specifically, it is known that perfume ingredients containing aldehyde functionality will react with the amine functionality of monomers used in capsule wall formation. This can result in the complete failure to encapsulate odourant oils containing aldehyde perfume ingredients, or if capsules are formed, the loading of odourant oil is low, and the capsules are susceptible to aggregation. Low odourant oil retention is costly, whereas aggregation phenomena are at the very least aesthetically undesirable and at worst can lead to manufacturing problems and poor capsule performance, and so should be avoided as much as possible.

WO 2011/161265 A2 proposed a solution to this problem, which consisted in presenting aldehyde perfume ingredients in the form of aldehyde precursors, in which the aldehyde functionality is protected and therefore unable to react with amine monomers during capsule formation. Whereas this is an interesting solution to the problem, nevertheless there is additional cost and complexity associated with preparing precursors of aldehyde perfume ingredients.

In a particular embodiment of the present invention, when the encapsulated perfume composition is employed to encapsulate aldehyde-containing perfume ingredients, in addition to said aldehyde perfume ingredients, the encapsulated perfume should contain a non-aromatic cyclic perfume ingredient, and an alkyl salicylate and/or a 2,2,2-trisubstituted acetal, wherein said acetal has the general formula

R₁R₂R₃C-CH(OR₄)(OR₅)

wherein R₁ is a saturated or unsaturated alkyl or aromatic residue having at least 4 carbon atom, more preferably at least 5 carbon atoms and most preferably at least 6 carbon atoms, but not more than 10 carbon atoms; R₂ and R₃ are independently selected from a saturated or unsaturated alkyl residue having at least on carbon atom; and R₄ and R₅ are independently selected from either a methyl group and/or an ethyl group.

In a more particular embodiment of the invention in addition to an aldehyde-containing perfume ingredient, the encapsulated perfume comprises a non-aromatic cyclic perfume ingredient and an alkyl salicylate.

In a more particular embodiment of the invention the encapsulated perfume comprises, in addition to an aldehyde-containing perfume ingredient, a non-aromatic cyclic perfume ingredient and an alkyl salicylate and a 2,2,2-trisubstituted acetal, hereinabove defined.

The term "cyclic perfume ingredient" as used herein refers to a molecule useful as a perfume ingredient, which contains within its chemical structure a series of atoms that forms a closed ring. That ring may be aromatic or aliphatic. It may be mono- or poly-cyclic, and it may contain hetero-atoms. The ring may bear substituents or it may be unsubstituted.

The aldehyde perfume ingredient may be any aldehyde useful in perfumery or as a flavourant. The skilled person in the art of perfumery has available to it a palette of ingredients containing aldehyde functionality, and these ingredients are contemplated in the present invention as representing aldehyde perfume ingredients. The aldehyde may be an aliphatic aldehyde, a cycloaliphatic aldehyde, and acyclic terpene aldehyde, a cyclic terpene aldehyde, or an aromatic aldehyde.

More particularly, the aldehydes include, but are not limited to, the following group of aldehydes, wherein CAS numbers are provided in parentheses:
DECANAL (112-31-2), 2-METHYL DECANAL (ALDEHYDE C-11 (19009-56-4), 10-UNDECEN-1-AL (112-45-8), UNDECANAL (112-44-7), DODECANAL (112-54-9), 2-METHYL UNDECANAL (110-41-8), HEPTANAL (111-71-7), OCTANAL (124-13-0), GREEN HEXANAL (5435-64-3), NONANAL (124-19-6), UNDECENAL MIXTURE (1337-83-3), (Z)-4-DECENAL (21662-09-9), (E)-4-DECENAL (65405-70-1), 9-DECENAL (39770-05-3), ISOVALERIANIC ALDEHYDE (590-86-3), AMYL CINNAMIC ALDEHYDE 122-40-7), METHYL CINNAMIC ALDEHYDE (101-39-3), METHYL PHENYL HEXENAL (21834-92-4), PHENYL PROPIONIC ALDEHYDE(104-53-0), PARA TOLYL ALDEHYDE (104-87-0), PARA ANISALDEHYDE (123-11-5), BENZALDEHYDE (100-52-7), CYCLAL C (68039-49-6), TRICYCLAL (68039-49-6), CYCLOMYRAL (68738-94-3), ISOCYCLOCITRAL (1335-66-6), MACEAL (68259-31-4), SAFRANAL (116-26-7), HELIOTROPINE (120-57-0), HEXYL CINNAMIC ALDEHYDE (101-86-0), BOURGEONAL (18127-01-0), CINNAMIC ALDEHYDE (104-55-2), CUMINIC ALDEHYDE (122-03-2), CYCLAMEN ALDEHYDE (103-95-7), CYCLOHEXAL (31906-04-4), FENNALDEHYDE (5462-06-6), FLORALOZONE (67634-15-5), FLORHYDRAL (125109-85-5), HYDRATROPIC ALDEHYDE (93-53-8), LILIAL (80-54-6), MEFRANAL (55066-49-4), MYRALDENE (37677-14-8), SILVIAL (6658-48-6), TRIFERNAL (16251-77-7), 2-TRIDECENAL (7774-82-5), DUPICAL (30168-23-1), SCENTENAL (86803-90-9), PRECYCLEMONE B (52475-86-2), VERNALDEHYDE (66327-54-6), HEXANAL (66-25-1), ADOXAL (141-13-9), CALYPSONE (929253-05-4), CETONAL (65405-84-7), CITRAL (5392-40-5), CITRONELLAL (106-23-0), CITRONELLYL OXYACETALDEHYDE (7492-67-3), DIHYDRO FARNESAL (32480-08-3), HYDROXYCITRONELLAL (107-75-5), MELONAL (106-72-9), METHOXYMELONAL (62439-41-2), NONADIENAL (557-48-2), ONCIDAL (54082-68-7), PINOACETALDEHYDE (33885-51-7), TETRAHYDRO CITRAL (5988-91-0), TROPIONAL (1205-17-0), ETHYL VANILLIN (121-32-4), VANILLIN (121-33-5).

When assigning perfume ingredients to categories, a perfume ingredient that contains both aldehyde functionality and a ring, is consider to be an aldehyde perfume ingredient for the purpose of the present invention, and not a cyclic perfume ingredient.

The extent of an aggregation phenomenon depends on a number of factors, including the reactivity of the aldehyde perfume ingredient towards monomers used in forming the capsule shells, e.g. an amine monomer, as well as the solubility of the aldehyde perfume ingredient in aqueous media. As the capsule shell-forming process is an interfacial process and the amines used are substantially contained in the aqueous phase, the extent to which an aldehyde perfume ingredient will partition into the aqueous phase, may affect its reactivity towards the amine.

In a particular embodiment of the present invention, the encapsulated perfume composition may contain up to 6 % by weight based on the total weight of the encapsulated perfume. More particularly, the encapsulated perfume composition contains encapsulated aldehyde perfume ingredients within the range of 0.01 % to 6 % by weight, more particularly still 0.01 to 5.5%, still more particularly 0.01 to 5 %, still more particularly 0.01 to 4.5 %, still more particularly 0.01 to 4.0 %, still more particularly 0.01 to 3.5 %, still more particularly 0.01 to 3%, still more particularly 0.01 to 2%, still more particularly 0.01 to 1 % by weight.

Non-aromatic cyclic perfume ingredients include, but are not limited to, cyclic esters, ketones, ketals and alcohols. Particularly useful non-aromatic cyclic perfume ingredients in the present invention are cyclic esters. Examples of useful cyclic esters include ACETYLATED CLOVE OIL TERPENES (68425-19-4), AGRUMEX (88-41-5), ALLYL CYCLOHEXYL PROPIONATE (2705-87-5), AMBER CORE (139504-68-0), AMBREINE (8016-26-0), AMBREINOL (73138-66-6), AMBRETTOLIDE (28645-51-4), AMBRINOL (41199-19-3), AMBROFIX (6790-58-5), APHERMATE (25225-08-5), AZARBRE (68845-36-3), BICYCLO NONALACTONE (4430-31-3), BOISIRIS (68845-00-1), BORNEOL (507-70-0), BORNYL ACETATE LIQUID (125-12-2), PARA BUTYL CYCLOHEXANOL (98-52-2), PARA BUTYL CYCLOHEXYL ACETATE (32210-23-4), CAMONAL (166301-22-0), CAMPHOR SYNTHETIC (76-22-2), LAEVO CARVONE (6485-40-1), CASHMERAN (33704-61-9), CEDRENE (11028-42-5), CEDRENOL (28231-03-0), CEDROL (77-53-2), WOODY EPOXIDE (71735-79-0), CEDRYL ACETATE CRYSTALS (77-54-3), CEDRYL METHYL ETHER (19870-74-7), CELERY KETONE (3720-16-9), CETALOX (3738-00-9), CIVETTONE (542-46-1), CONIFERAN (67874-72-0), CORANOL (83926-73-2), COSMONE (259854-70-1), CYCLOGALBANATE (68901-15-5), CYCLOHEXYL ETHYL ACETATE (21722-83-8), CYPRISATE (23250-42-2), DAMASCENONE (23696-85-7), ALPHA DAMASCONE (24720-09-0), BETA DAMASCONE (23726-92-3), DELTA DAMASCONE (57378-68-4), DELTA DECALACTONE (705-86-2), GAMMA DECALACTONE (706-14-9), DECATONE (34131-98-1), DIHYDRO AMBRATE (37172-02-4), BETA DIHYDRO IONONE (17283-81-7), DIHYDRO JASMONE (1128-08-1), DELTA DODECALACTONE (713-95-1), DODECALACTONE GAMMA (2305-05-7), DUPICAL (30168-23-1), ETHYL SAFRANATE (35044-59-8), ETHYLENE BRASSYLATE (105-95-3), EUCALYPTOL (470-82-6), ALPHA FENCHONE (7787-20-4), FENCHYL ACETATE (13851-11-1), FENCHYL ALCOHOL (1632-73-1), FLOROCYCLENE (68912-13-0), FLOROSA (63500-71-0), FLORYMOSS (681433-04-5), FOLENOX (26619-69-2), FOLROSIA (4621-04-9), FRESKOMENTHE (14765-30-1), FRUITATE (80623-07-0), GALBANONE PURE (56973-85-4), GARDOCYCLENE (67634-20-2), GEORGYWOOD (185429-83-8), GIVESCONE (57934-97-1), GLYCOLIERRAL (68901-32-6), GRISALVA (68611-23-4), GYRANE (24237-00-1), HABANOLIDE (111879-80-2), HEDIONE (24851-98-7), HEPTALACTONE GAMMA (105-21-5), HERBANATE (116126-82-0), HERBAVERT (67583-77-1), HERBOXANE (54546-26-8), BETA IONONE (8013-90-9), IRISANTHEME (1335-46-2), ALPHA IRISONE (8013-90-9), ALPHA IRONE (79-69-6), IRONE F (54992-91-5), ISO E SUPER (54464-57-2), ISOJASMONE B 11 (95-41-0), ISOLONGIFOLANONE (23787-90-8), ISOMENTHONE DL (491-07-6), ISOPULEGOL (89-79-2), ISORALDEINE 40, 70 and 90 (1335-46-2), JASMACYCLENE (5413-60-5), JASMATONE (13074-65-2), JASMOLACTONE (32764-98-0), CIS JASMONE (488-10-8), JASMONYL (18871-14-2), KARANAL (117933-89-8), KEPHALIS (36306-87-3), LAITONE (4625-90-5), LIGANTRAAL (68738-99-8), MAYOL (13828-37-0), MENTHONE (89-80-5), METAMBRATE (72183-75-6), METHYL CEDRYL KETONE (32388-55-9), GAMMA METHYL DECALACTONE (7011-83-8), METHYL DIHYDRO ISOJASMONATE (37172-53-5), METHYL EPI JASMONATE (39924-52-2), METHYL TUBERATE (33673-62-0), MUSCENONE (82356-51-2), MUSCONE (541-91-3), ETHYLENE DODECANOATE (54982-83-1), MUSK LACTONE (3391-83-1), MYRALDYL ACETATE (72403-67-9), NECTARYL (95962-14-4), NIMBEROL (70788-30-6), NIRVANOLIDE (329925-33-9), NOOTKATONE (4674-50-4), NOPYL ACETATE (128-51-8), DELTA OCTALACTONE (698-76-0), GAMMA OCTALACTONE (104-50-7), OKOUMAL (131812-67-4), OPALAL (62406-73-9), ORIVONE (16587-71-6), OXYOCTALINE FORMATE (65405-72-3), PIVACYCLENE (68039-44-1), PLICATONE (41724-19-0), POIRENATE (2511-00-4), QUINTONE (4819-67-4), RHUBOFIX (41816-03-9), RHUBOFLOR (93939-86-7), ROSE OXIDE CO (16409-43-1), ROSE OXIDE LAEVO (3033-23-6), ROSSITOL (215231-33-7), SAFRALEINE (54440-17-4), SANDELA (66068-84-6), SPIRAMBRENE (121251-67-0), SPIROGALBANONE (224031-70-3), SUPERFIX (3910-35-8), THIBETOLIDE (106-02-5), TIMBEROL (70788-30-6), TRIMOFIX O (144020-22-4), DELTA UNDECALACTONE (710-04-3), GAMMA VALEROLACTONE (108-29-2), VELOUTONE (65443-14-3), VELVIONE (37609-25-9), VERDALIA (27135-90-6), VERDOL (13491-79-7), VERTOFIX COEUR (32388-55-9), VETIKOL ACETATE (68083-58-9), VETIVERYL ACETATE (68917-34-0), VETYNAL (57082-24-3). Useful alkyl salicylates include AMYL SALICYLATE (2050-08-0), ETHYL SALICYLATE (118-61-6), HEXENYL-3-CIS SALICYLATE (65405-77-8), HEXYL SALICYCLATE (6259-76-3), ISOBUTYL SALICYLATE (87-19-4), ISOBUTYL SALICYLATE (87-19-4), KARMAFLOR (873888-84-7), METHYL SALICYLATE (119-36-8).

Useful 2,2,2-substituted acetals include METHYL PAMPLEMOUSSE (67674-46-8), AMAROCIT B (72727-59-4), NEROLIACETAL (99509-41-8).

The non-aromatic cyclic perfume ingredients and alkyl salicylates, independently of each other may be present in amounts of 10 % or greater by weight based on the total weight of encapsulated perfume, more particularly 15 % or greater, more particularly 20 % or greater, more particularly 25 % or greater, still more particularly 30 % or greater, more particularly 33% or greater, for example 20 to 99.99%, or 25 to 99.99%,or 25 to 99.99%, or 30 to 99.99%, or 33 to 99.99% .

In a particular embodiment of the present invention the aldehyde perfume ingredients are present in an amount of 1% to 6 % by weight, more particularly 2% to 5.5 % by weight, still more particularly 3% to 5 % by weight; and the non-aromatic cyclic perfume ingredients and/or alkyl salicylates perfume ingredients are independently present in amounts of more than 30 % by weight, still more particularly more than 33 % by weight.

In another particular embodiment of the present invention the aldehyde perfume ingredients are present in an amount of 1% to 6 % by weight, more particularly 2% to 5.5 % by weight, still more particularly 3% to 5 % by weight; the non-aromatic cyclic perfume ingredients and/or alkyl salicylates perfume ingredients independently are present in amounts between 10% and 33% by weight.

In yet another particular embodiment of the invention the aldehyde perfume ingredients are present in an amount of 1% to 6 % by weight, more particularly 2% to 5.5 % by weight, still more particularly 3% to 5 % by weight; the non-aromatic cyclic perfume ingredients and alkyl salicylates perfume ingredients independently are present in amounts between 10% and 33% by weight and the 2,2,2-substituted acetals are present in amounts of more than 25% by weight, more particularly more than 30% by weight, still more particularly more than 33% by weight.

The encapsulated perfume compositions according to the present invention may be incorporated as slurries into consumer products. However, it may also be desired to incorporate encapsulated perfume compositions in the form of a dry powder.

A method of dehydrating an encapsulated perfume composition, as well as the resultant encapsulated perfume composition in powdered form, represent additional aspects of the present invention.

The slurry may be dried using techniques known in the art. For example, it may be dried by decanting off the liquid from the suspension and drying the capsules in an oven to produce a cake, which can then be rendered in powder form by a subsequent comminution step.

Preferably, however, drying of the slurry is carried out by spray drying or fluid-bed drying, without further handling.

Spray drying techniques and apparatus are well known in the art. A spray-drying process pushes suspended capsules through a nozzle and into a drying chamber. The capsules may be entrained in a fluid (such as air) that moves inside of a drying chamber. The fluid (which may be heated, for example at a temperature of 150 and 120 °C, more preferably between 170 °C and 200 °C, and still more preferably between 175 °C and 185 °C.) causes the liquid to evaporate, leaving behind the dried capsules, which can then be collected from the process equipment, and further processed.

It is conventional to mix spray dried capsules with flow aids to produce a flowable powders that are not susceptible to caking. Flow aids include silica or silicates, such as precipitated, fumed or colloidal silica; starches; calcium carbonate; sodium sulphate; modified cellulose; zeolites; or other inorganic particulates known in the art.

It is quite common, given the high temperatures and impaction forces encountered during a spray drying procedure, for core shell capsules to lose some of their core material. Furthermore, it may not be possible to work at sufficiently high temperatures for a sufficiently long period of time to drive off all moisture from the slurry, without compromising the thermal stability of the capsules. Accordingly, the polyurea capsules emerging from a spray-drying process as herein described, may contain small amounts of surface oil, as well as residual moisture. Applicant found, however, that the conventional use of flow aids, added to the dried capsules, was not completely effective to produce the polyurea capsules of the present invention in a free-flowing form that was not prone to caking.

Surprisingly, however, applicant found that if the flow aid was added to the slurry before the spray-drying step, the resultant polyurea capsules produced fine, free-flowing powders that did not cake or show any signs of agglomeration.

More particularly, the applicant found that particularly good powders were formed that were free-flowing, resistant to caking, and had low levels of residual moisture and surface oil, when the flow-aid added to the slurry was a form of silica having a volume average particle size that was micron-sized, and more particularly from 1 to 8 microns, still more particularly from 1 to 7, more particularly from 1 to 6, and still more particularly from 1 to 5 microns.

Still further, the applicant found that employing said silica having a bulk density of 5 to 30 lbs/ft³ resulted in particularly good powders that were free-flowing, resistant to caking, and had low levels of residual moisture and surface oil.

Syloid FP grade silicas were particularly preferred flow aids, for example Syloid FP 244, Syloid FP 72, or Syloid FP 63.

Accordingly, the invention provides in another of its aspects a method of making an encapsulated perfume composition as herein defined, in the form of a powder, comprising the step of spray-drying a slurry comprising a plurality of polyurea capsules as herein defined, dispersed in an aqueous medium comprising a silica flow aid as herein above defined.

In another aspect of the present invention there is provided an encapsulated perfume composition as herein defined, in the form of a powder comprising a flow aid as hereinabove described, said powder having a residual moisture content of 0.1 to 8 % by weight, more particularly 0.5 to 5 % by weight, and still more particularly 1 to 3 % by weight, based on the weight of the slurry.

In yet another aspect of the present invention there is provided an encapsulated perfume composition as herein defined, in the form of a powder comprising a flow aid as hereinabove described, said powder having a surface oil (oil lost from the core) content of less than 5 %, more particularly less than 4 %, and still more particularly less than 0.5 % based on the weight of the powder.

Residual moisture can be measured using the Karl Fisher method, whereas the amount of surface oil can be measured by extracting the powder with a solvent for the oil, and analysing using GC MS.

The present invention also relates to the incorporation of an encapsulated perfume composition as hereinabove defined into all manner of personal care and household care products. Particular categories of products include personal care products, and in particular those products adapted to be applied to and left on the skin or hair of a subject. The present invention also relates to a personal care or household care product containing an encapsulated perfume composition as hereinabove defined.

The encapsulated perfume composition according to the present invention may be incorporated into said products as a slurry or a powder.

The level of incorporation of an encapsulated perfume composition into consumer products will vary depending on the product to be perfumed and the effect that needs to be achieved. Typically, the capsules may form between 0.01 to 50% by weight of a consumer product containing same, most preferably from 0.1% to 2% by weight of a consumer product containing same.

The encapsulated perfume composition of the present invention may be the sole source of perfume material incorporated into said products. However, additional perfume may also be incorporated into said products in the form of free (un-encapsulated) perfume, or other types of encapsulated perfume compositions may be employed with the encapsulated perfume composition of the present invention. Other types of encapsulated perfume compositions may include any capsules known to contain perfume, such as gelatin capsules, starch capsules, acrylic capsules, aminoplast capsules, and the like. The other capsule types may release their perfume by diffusion, or by any external physical stimulus, such as heat, moisture, light, or by abrasion.

In yet another aspect of the invention there is provided a method to confer, enhance, improve or modify the olfactive properties of a personal care or household care product, and in particular a leave-on product, which method comprises incorporating into said product an encapsulated perfume composition as hereinabove defined.

The provision of deodorant and antiperspirant products, containing an encapsulated perfume composition as hereinabove defined, which reliably releases perfume when subjected to shear forces, such as the frictional force of skin against human or animal skin or skin against an inanimate surface such as a textile, and does so over a period of time up to 6 hours, and more preferably up to 10 hours, addresses an unmet need.

Accordingly, in another aspect of the invention there is provided the use of an encapsulated perfume composition as described herein, to perfume a consumer product, in particular a household, or personal care product. The compositions of the present invention are particularly suitable for use in leave-on applications, such as cosmetic creams and lotions, or deodorant formulations and antiperspirant formulations.

In an embodiment of the present invention there is provided a personal care product for perfuming human or animal skin or hair comprising an encapsulated perfume composition as hereinabove defined.

In an embodiment of the present invention there is provided a personal care product for perfuming human or animal skin or hair comprising an encapsulated perfume composition as hereinabove defined, which is a rinse-off or leave-on product.

In an embodiment of the invention the leave-on product may be a deodorant, for example an under arm deodorant such as a roll-on or stick deodorant or an antiperspirant aerosol spray, or a body lotion, or body spray, or cream, or a hair cream such as a combing cream, or talcum powder.

In an embodiment of the present invention the rinse-off product may be a shower gel, solid or liquid soap, a shampoo or a conditioner.

Furthermore, the encapsulated perfume composition of the present invention can be used in all the fields of modern perfumery to positively impart or modify the odour of a product into which said compositions are added. The nature and type of the constituents of a perfumed product do not warrant a more detailed description here, which in any case would not be exhaustive, the skilled person being able to select them on the basis of its general knowledge and according to the nature and the desired effect of said product.

Examples of suitable products include perfumed soaps, shower or bath salts, mousses, oils or gels, hygiene products or hair care products such as shampoos, body-care products, deodorants and antiperspirants.

In a particular aspect of the present invention, the encapsulated perfume compositions are incorporated into an anti-perspirant and/or deodorant roll-on, stick or aerosol personal care products. The anti-perspirant and/or deodorant personal care products contain an effective amount of the capsules. In addition to comprising capsules according to invention, the anti-perspirant and/or deodorant aspect of the invention may comprise at least one deodorant active principle and/or at least one anti-perspirant salt or complex.

Within the meaning of the instant invention, "deodorant active principle" is understood to mean any substance capable of masking, absorbing, improving or reducing the unpleasant odour resulting from the decomposition of human sweat by bacteria.

More specifically, the deodorant active principles can be bacteriostatic agents or bactericidal agents, such as 2,4,4'-trichloro-2'-hydroxydiphenyl ether ((R)-Triclosan), 2,4-dichloro-2'-hydroxydiphenyl ether, 3',4',5 '-trichlorosalicylanilide, 1-(3',4'-dichloro-phenyl)-3-(4'-chlorophenyl)urea ((R)-Triclocarban) or 3,7,11-trimethyldodeca-2,5,10-trienol ((R)-Farnesol); quaternary ammonium salts, such as cetyltrimethyl-ammonium salts or cetylpyridinium salts, DPTA (1,3-diaminopropanetetraacetic acid) or 1,2-decanediol (Simclariol from Symrise).

Mention may also be made, among the deodorant active principles of zinc salts, such as zinc salicylate, zinc gluconate, zinc pidolate, zinc sulphate, zinc chloride, zinc lactate or zinc phenoisulphonate; chlorhexidine and its salts; sodium bicarbonate; salicylic acid and its derivatives, such as 5-(n-octanoyl)salicylic acid; glycerol derivatives, such as, for example, caprylic/capric glycerides (Capmul MCM from Abitec), glycerol caprylate or caprate (Dermosoft GMCY and Dermosoft GMC respectively from Straetmans) or polyglyceryl-2 caprate (Dermosoft DGMC from Straetmans); biguanide derivatives, such as polyhexamethylene-biguanide salts; silver, zeolites or silver-free zeolites.

In order to improve the antiperspirant effectiveness of the composition, use may additionally be made of one or more water-soluble anionic polymers comprising a Bronsted acid, in particular those deriving from maleic acid and/or maleic anhydride which are described in Patent Application WO 02/49590 A2.

Furthermore, "anti-perspirant salt or complex" as herein refers to any salt or complex which, by itself alone, has the effect of reducing or limiting the flow of sweat and/or absorbing human sweat. Examples of such anti-perspirant salt or complexes can be found in the OTC final monograph on antiperspirant actives and in US 2010/0196484 A1, US 2005/0031565 A1, US 2005/0238598 A1, and US 2011/0212144 A1.

The antiperspirant salts or complexes are generally chosen from aluminium and/or zirconium salts or complexes. They are typically chosen from aluminium hydrohalides; aluminium zirconium hydrohalides, or complexes of zirconium hydroxychloride and of aluminium hydroxychloride, with or without an amino acid, such as those described in U.S. 3,792,068.

Mention may in particular be made, among the aluminium salts, of aluminium chlorohydrate in the activated or nonactivated form, aluminium chlorohydrex, the aluminium chlorohydrex polyethylene glycol complex, the aluminium chlorohydrex propylene glycol complex, aluminium dichlorohydrate, the aluminium dichlorohydrex polyethylene glycol complex, the aluminium dichlorohydrex propylene glycol complex, aluminium sesquichlorohydrate, the aluminium sesquichlorohydrex polyethylene glycol complex, the aluminium sesquichlorohydrex propylene glycol complex or aluminium sulphate buffered with sodium aluminium lactate.

Mention may in particular be made, among aluminium zirconium salts, of aluminium zirconium octachlorohydrate, aluminium zirconium pentachlorohydrate, aluminium zirconium tetrachlorohydrate or aluminium zirconium trichlorohydrate.

The complexes of zirconium hydroxychloride and of aluminium hydroxychloride with an amino acid are generally known under the name ZAG (when the amino acid is glycine). Mention may be made, among these products, of the aluminium zirconium octachlorohydrex glycine, aluminium zirconium pentachlorohydrex glycine, aluminium zirconium tetrathiorohydrex glycine and aluminium zirconium trichlorohydrex glycine complexes.

In order to further illustrate the present invention and the advantages thereof, the following specific examples and comparative example are given, it being understood that same are intended only as illustrative and in nowise limitative.

### Example 1

Microcapsules were prepared as follows:
A premix (I) comprises 25 g Polyvinylpyrolidone K60 and 650g water was prepared and the pH was adjusted to 10.0 using sodium hydroxide solution. Premix (II) comprises 300 g perfume to be encapsulated, 20 g Desmodur® W and 5 g Bayhydur® XP 2547 was prepared.

The two premixes were combined and emulsified at room temperature by means of a stirring device. The emulsification process was carried out to the desired droplet size. The pH of the emulsion was then adjusted to 8 using aqueous sodium hydroxide solution. Then 10 g of Lupasol® PR8515 solution was added in one step.

The reaction mixture was heated until the initiation was initiated.

The mixture was then cooled down to room temperature.

An encapsulated perfume composition was obtained. The volume average capsule size distribution, obtained with light scattering measurements using a Malvern 2000S instrument, was D50 = 20 microns and D 90 = 50 microns with a shell weight 6% of total slurry weight composition. The solid content of the slurry was 40 weight %.

### Example 2

Encapsulated perfume compositions were prepared according to the methodology set forth in Example 1. The compositions contained 25% by weight of slurry of perfume compositions having ingredients specified in the Tables 1 through 5, below. The encapsulation process was described in Example 1 above. The amounts of aldehydes, non-aromatic cyclic perfumer ingredients and alkyl salicylates contained in the perfumes are shown (parts by weight of the perfume). The balance of the perfume is formed from other perfume ingredients commonly used in perfumery.

The compositions of the perfumes used in the example are listed in Tables 1 to 5. Under "ionone family" is meant ionones, irones, isoraldeines, damascones, damascenone, galbanone, and the like.

**Table 1: Perfume 1 composition**

| | Other ingredients | Non-aromatic cyclic ingredients | Alkyl salicyclates | Aldehydes |
|---|---|---|---|---|
| AROMATIC ESTERS | 3 | | | |
| NON-CYCLIC NON-AROMATIC ESTERS | 7 | | | |
| ALKYL CARBONATES | 1.5 | | | |
| DIMETHYL BENZYL CARBINYL ACETATE | 2 | | | |
| AGRUMEX | | 5 | | |
| PARA-ANISALDEHYDE | | | | 0.3 |
| TERPENE ALCOHOLS | 22 | | | |
| TERPINEOL | | 2 | | |
| TERPENYL ACETATE | | 2 | | |
| CITRONELLYL NITRILE | 1 | | | |
| IONONE FAMILY | | 10.7 | | |
| EUCALYPTOL | | 0.8 | | |
| FLOROSA | | 5 | | |
| GARDOCYCLENE | | 1 | | |
| INDOFLOR | 0.3 | | | |
| ISO E SUPER | | 10 | | |
| JASMONE FAMILY | | 1 | | |
| MAYOL | | 2 | | |
| AROMATIC ALCOHOL | 5 | | | |
| MENTHONE | | 0.3 | | |
| LACTONES | | 0.5 | | |
| HEXYL SALICYCLATE | | | 10 | |
| RADJANOL | 2 | | | |
| AROMATIC ETHERS | 0.3 | | | |
| ROSE OXIDE | | 0.3 | | |
| MACROCYCLIC MUSKS | | 5 | | |
| TOTAL | 44.1 | 45.6 | 10 | 0.3 |

**Table 2: Perfume 2 composition**

| | Other ingredients | Non-aromatic cyclic ingredients | Alkyl salicyclates | Aldehydes |
|---|---|---|---|---|
| AROMATIC ESTERS | 3 | | | |
| NON-CYCLIC NON-AROMATIC ESTERS | 8 | | | |
| ALKYL CARBONATES | 3 | | | |
| BORNYL ACETATE | | 3 | | |
| ALDEHYDE C 12 MNA | | | | 1 |
| FLORALOZONE | | | | 1 |
| TERPENE ALCOHOLS | 37 | | | |
| KETALS | 5 | | | |
| LEMONILE | 0 | | | |
| IONONE FAMILY | | 3 | | |
| CAMPHRE | | 2 | | |
| PHENOLS | 0 | | | |
| JASMACYCLENE | | 2 | | |
| ISO E SUPER | | 10 | | |
| AROMATIC ALCOHOL | 4 | | | |
| CIS-3-HEXENYL | | | | |
| SALICYLATE | | | 3 | |
| HEXYL SALICYCLATE | | | 10 | |
| AROMATIC ETHERS | 0 | | | |
| MACRO CYCLIC | | | | |
| MUSKS | | 5 | | |
| TOTAL | 59 | 26 | 13 | 2 |

**Table 3: Perfume 3 composition**

| | Other ingredients | Non-aromatic cyclic ingredients | Alkyl salicyclates | Aldehydes |
|---|---|---|---|---|
| AROMATIC ESTERS | 8 | | | |
| NON-CYCLIC NON-AROMATIC ESTERS | 15 | | | |
| ALKYL CARBONATES | 2 | | | |
| PARA TERT BUTYL CYCLOHEXYL ACETATE | | 5 | | |
| AGRUMEX | | 8 | | |
| TERPENE ALCOHOLS | 11 | | | |
| FLORHYDRAL | | | | 2 |
| HELIOTROPINE | | | | 1 |
| IONONE FAMILY | | 8 | | |
| FLOROCYCLENE & HERBANATE | | 6 | | |
| INDOFLOR ISO E SUPER | | 4 | | |
| JASMONE FAMILY | | 2 | | |
| AROMATIC ALCOHOL | 1 | | | |
| LACTONES | | 5 | | |
| MACROCYCLIC | | 5 | | |
| MUSKS PHENOLS | 0.2 | | | |
| HEDIONE | | 16 | | |
| NECTARYL | | 2 | | |
| TOTAL | 38 | 60 | 0 | 2 |

**Table 4: Perfume 4 composition**

| | Other ingredients | Non-aromatic cyclic ingredients | Alkyl salicyclates | Aldehydes |
|---|---|---|---|---|
| NON-CYCLIC NON-AROMATIC ESTERS | 16.0 | | | |
| ALLYL CYCLOHEXYL PROPIONATE | | 2.0 | | |
| AGRUMEX | | 35.4 | | |
| ALCOHOLS | 3.0 | | | |
| LILIAL | | | | 5.0 |
| IONONE FAMILY | | 1.1 | | |
| JASMACYCLENE | | 20.0 | | |
| LACTONES | | 10.0 | | |
| CIS-3-HEXENYL SALICYLATE | | | 2.0 | |
| NECTARYL | | 5.0 | | |
| TOTAL | 19.0 | 73.5 | 2.0 | 5.0 |

**Table 5: Perfume 5 composition**

| | Other ingredients | Non-aromatic cyclic ingredients | Alkyl salicyclates | Aldehydes | Acetals (1) |
|---|---|---|---|---|---|
| AROMATIC ESTERS | 3.4 | | | | |
| NON-CYCLIC NON-AROMATIC ESTERS | 6.0 | | | | |
| ALKYL CARBONATES | 4.8 | | | | |
| DIMETHYL CARBINYL ACETATE | | | 6.0 | | |
| ALDEHYDE C 12 MNA | | | | 0.7 | |
| FLORALOZONE | | | | 1.4 | |
| TERPENE ALCOHOLS | 43.4 | | | | |
| METHYL PAMPLEMEOUSSE | | | | | 12.0 |
| CITRONELLYL NITRILE | 2.4 | | | | |
| LEMONILE | 0.2 | | | | |
| BORNYL ACETATE | | 2.4 | | | |
| INDOFLOR ISO E SUPER | | 12.0 | | | |
| CAMPHRE | | 1.7 | | | |
| SYLKOLIDE | 1.0 | | | | |
| AROMATIC ETHERS | 0.4 | | | | |
| MACRO CYCLIC MUSKS | 0.5 | | | | |
| MINOR COMPONENTS | 1.4 | | | | |
| TOTAL | 63.6 | 16.1 | 6.0 | 2.2 | 12.0 |

| | | | | | |
|---|---|---|---|---|---|
| (1) 2,2,2-trisubstituted acetals | | | | | |

**Table 6: Encapsulation performance of the perfume compositions**

| | Aldehydes | Non-aromatic cyclic ingredients | Salicylates | 2,2,2-trisubstituted acetals | Encapsulation |
|---|---|---|---|---|---|
| Perfume 1 | 0.3 | 45.6 | 10 | | YES |
| Perfume 2 | 2.0 | 26.0 | 13.0 | | YES |
| Perfume 3 | 2.0 | 60.0 | 0 | | YES |
| Perfume 4 | 5.5 | 73.5 | 2.0 | | YES |
| Perfume 5 | 2.2 | 16.1 | 6.0 | 12.0 | YES |

### Example 3

A sensory test was carried out to compare the intensity of two samples of encapsulated perfume composition, formed according to the method of Example 1, containing the same perfume but of two different sizes with D50 of 10 and 30 microns, overtime when in a roll-on deodorant base. The roll-on deodorants were tested on skin by a trained sensory panel. The products were assessed when freshly applied and then 2 hours, 6 hours and 10 hours after application. After 10 hours the products were also assessed after rubbing and directly from the skin.

The overall perceived intensity was assessed by the trained sensory panel using a 0-100 scale.

The panelists were instructed to smell their underarm immediately after sample application and then after 2 hours, 6 hours, 10 hours and 10 hours post rub through the T-shirt. 10 hours after application and after rub the under arms were also assessed directly from the skin.

For the rubbing assessment the panelists were instructed to move their left arm forward and their right arm backwards simultaneously whilst ensuring the upper arm rubs the side of their body and their lower arm is horizontally out in front of them. They were asked to make this movement four times in total.

Allocation of which sample was applied to which arm (left or right) was carried out according to a predetermined randomization and the panelists were always asked to assess their left underarm first. Each sample was assessed once by 21 panelists.

The data were analyzed using a Student T-test. The confidence level was 95%.

**Table 7**

| Capsule Diameter | Shell Weight (1) (%) | Time 0 Initial | Time 2 hours | Time 6 hours | Time 10 hours | Time 10 hours Post-rub |
|---|---|---|---|---|---|---|
| D50 = 10 microns | 15 | 28 | 22 | 19 | 13 | 18 |
| D50 = 30 microns | 15 | 38 | 30 | 23 | 13 | 20 |
| D50 = 30 microns | 19 | 37 | 27 | 23 | 14 | 20 |
| D50 = 30 microns | 23 | 28 | 23 | 20 | 13 | 18 |

| | | | | | | |
|---|---|---|---|---|---|---|
| (1) Percentage by weight based on the capsule weight (encapsulated material + shell material) | | | | | | |

The results show a significant benefit of the capsules having a shell weight to diameter ratio of less than 0.7.

### Example 4

A series of slurries containing polyurea capsules were formulated as disclosed in Table 8 and the extent of phase separation was measured after 1week at 50°C. As apparent from the results, no phase separation is observed when using hydroxyethyl cellulose (Natrosol 250HX) at 0.4% by weight, and the slurry remains pourable. All other dispersion aids fail to stabilize the slurry over the test period.
Phase separation was measured by naked eye assessment and was expressed as the ratio of the height of the water phase to the total height of the slurry.

**Table 8**

| | 1 % | 2 % | 3 % | 4 % | 5 % | 6 % | Natrosol 250 HX (wt %) | Phase separation % | Viscosity (cps) |
|---|---|---|---|---|---|---|---|---|---|
| Slurry A | | | | | | 1.5 | 0 | 40 | |
| Slurry B | | | | | | | 0.4 | 0 | 2400 |
| Slurry C | 3 | | | | | | 0 | 10 | |
| Slurry D | | 3.5 | | | | | 0 | 10 | |
| Slurry E | | | 1.5 | | | | 0 | 15 | |
| Slurry F | | | | 0.5 | | | 0 | 30 | |
| Slurry G | | | | | 2 | | 0 | 40 | |

| | | | | | | | | | |
|---|---|---|---|---|---|---|---|---|---|
| 1 = National 465; 2 = Starch B990; 3 = Tinovis CD; 4 = Ultragel 300; 5 = Rheocare TTA; 6 = Purity W | | | | | | | | | |

### Example 5

90g of an encapsulated perfume composition formed according to the procedure of example 1 was formed as a slurry. To this slurry was added 9g of Capsul E (@ 23% in water) and 1g of silica (Syloid FP 244). The slurry was agitated 30min at 250 rpm and spray dried in a spray dryer (labplant) using an atomizer. The inlet temperature was 180 °C and the outlet temperature was 90 °C. A free flowing powder was obtained with a D50 of 30 microns and 65% fragrance loading. The residual water constant was 4% by weight and the surface oil was 0.8% by weight.

## Claims

1. An encapsulated perfume composition in the form of a slurry comprising one or more core-shell capsules, dispersed in an aqueous dispersing medium, wherein the core contains a perfume and the shell contains a polyurea resin, and wherein the capsules are in the form of a stable suspension having a viscosity of up to 3000 mPa·s, and more particularly 150 to 3000 mPa·s when measured on a rheometer, using rotating disks at a shear rate of 21 s⁻¹ at a temperature of 25 °C, and wherein the aqueous dispersing medium contains a hydroxyethyl cellulose dispersing aid.

2. An encapsulated perfume composition according to claim 1 wherein the hydroxyethyl cellulose is present in amounts of 0.05 to 1.0 % based on the total weight of the slurry.

3. An encapsulated perfume composition according to claim 1 or claim 2 wherein the hydroxyethyl cellulose is a Natrosol™ hydroxyethyl cellulose.

4. An encapsulated perfume composition according to any of the preceding claims wherein the polyurea capsules have a volume average diameter of 20 to 250 microns.

5. An encapsulated perfume composition according to any of the preceding claims wherein the weight of the capsule shells is 5 % to 40 % based on the total weight of the capsules.

6. An encapsulated perfume composition according to any of the preceding claims wherein the encapsulated perfume comprises at least 60 % by weight of perfume ingredients having a solubility in water of 15'000 ppm or less.

7. An encapsulated perfume composition according to any of the preceding claims wherein the capsules contain no encapsulated solvent.

8. A consumer product comprising an encapsulated perfume composition as defined in any of the claims 1 through 7.

9. A consumer product according to claim 8, which is a leave-on personal care product.

10. A leave-on product according to claim 8 or 9 in the form of a deodorant product.

11. A leave on product according to claim 8 or 9 in the form of an anti-perspirant.

## Patentansprüche

1. Verkapselte Parfümzusammensetzung in Form einer Aufschlämmung, die eine oder mehrere in einem wässrigen Dispergiermedium dispergierte Kern-Schale-Kapseln umfasst, wobei der Kern ein Parfüm umfasst und die Schale ein Polyharnstoffharz umfasst und wobei die Kapseln in Form einer stabilen Suspension mit einer Viskosität von bis zu 3000 mPa·s und spezieller 150 bis 3000 mPa·s bei Messung auf einem Rheometer unter Verwendung von rotierenden Scheiben bei einer Scherrate von 21 s⁻¹ bei einer Temperatur von 25 °C vorliegen und wobei das wässrige Dispergiermedium eine Hydroxyethylcellulose-Dispergierhilfe enthält.

2. Verkapselte Parfümzusammensetzung nach Anspruch 1, wobei die Hydroxyethylcellulose in Mengen von 0,05 bis 1,0 %, bezogen auf das Gesamtgewicht der Aufschlämmung, vorliegt.

3. Verkapselte Parfümzusammensetzung nach Anspruch 1 oder Anspruch 2, wobei es sich bei der Hydroxyethylcellulose um eine Natrosol™-Hydroxyethyl-cellulose handelt.

4. Verkapselte Parfümzusammensetzung nach einem der vorhergehenden Ansprüche, wobei die Polyharnstoff-Kapseln einen volumenmittleren Durchmesser von 20 bis 250 Mikron aufweisen.

5. Verkapselte Parfümzusammensetzung nach einem der vorhergehenden Ansprüche, wobei das Gewicht der Kapselschalen 5 % bis 40 %, bezogen auf das Gesamtgewicht der Kapseln, beträgt.

6. Verkapselte Parfümzusammensetzung nach einem der vorhergehenden Ansprüche, wobei das verkapselte Parfüm mindestens 60 Gew.-% Parfümbestandteile mit einer Wasserlöslichkeit von 15.000 ppm oder weniger umfasst.

7. Verkapselte Parfümzusammensetzung nach einem der vorhergehenden Ansprüche, wobei die Kapseln kein verkapseltes Lösungsmittel enthalten.

8. Konsumprodukt, umfassend eine verkapselte Parfümzusammensetzung gemäß einem der Ansprüche 1 bis 7.

9. Konsumprodukt nach Anspruch 8, bei dem es sich um ein Leave-on-Körperpflegeprodukt handelt.

10. Leave-on-Produkt nach Anspruch 8 oder 9 in Form eines Deodorant-Produkts.

11. Leave-on-Produkt nach Anspruch 8 oder 9 in Form eines Antiperspirants.

## Revendications

1. Composition de parfum encapsulé sous la forme d'une suspension épaisse comprenant une ou plusieurs capsules à cœur-coquille, dispersées dans un milieu de dispersion aqueux, dans laquelle le cœur contient un parfum et la coquille contient une résine de polyurée et dans laquelle les capsules sont sous la forme d'une suspension stable ayant une viscosité allant jusqu'à 3000 mPa·s, et plus particulièrement de 150 à 3000 mPa·s lorsqu'elle est mesurée sur un rhéomètre, à l'aide de disques rotatifs à un taux de cisaillement de 21 s⁻¹ à une température de 25 °C, et dans laquelle le milieu de dispersion aqueux contient un adjuvant de dispersion hydroxyéthylcellulose.

2. Composition de parfum encapsulé selon la revendication 1 dans laquelle l'hydroxyéthylcellulose est présente en quantités de 0,05 à 1,0 % par rapport au poids total de la suspension épaisse.

3. Composition de parfum encapsulé selon la revendication 1 ou la revendication 2 dans laquelle l'hydroxyéthylcellulose est une hydroxyéthylcellulose Natrosol™.

4. Composition de parfum encapsulé selon l'une quelconque des revendications précédentes dans laquelle les capsules de polyurée ont un diamètre moyen en volume de 20 à 250 micromètres.

5. Composition de parfum encapsulé selon l'une quelconque des revendications précédentes dans laquelle le poids des coquilles des capsules est de 5 % à 40 % par rapport au poids total des capsules.

6. Composition de parfum encapsulé selon l'une quelconque des revendications précédentes dans laquelle le parfum encapsulé comprend au moins 60 % en poids d'ingrédients de parfum ayant une solubilité dans l'eau inférieure ou égale à 15 000 ppm.

7. Composition de parfum encapsulé selon l'une quelconque des revendications précédentes dans laquelle les capsules ne contiennent pas de solvant encapsulé.

8. Produit de consommation comprenant une composition de parfum encapsulé telle que définie dans l'une quelconque des revendications 1 à 7.

9. Produit de consommation selon la revendication 8, qui est un produit de soins personnels sans rinçage.

10. Produit sans rinçage selon la revendication 8 ou 9 sous la forme d'un produit déodorant.

11. Produit sans rinçage selon la revendication 8 ou 9 sous la forme d'un anti-transpirant.
